# EUROPÄISCHE PATENTSCHRIFT

(11) **EP 1 905 369 B1**
(45) Veröffentlichungstag und Bekanntmachung des Hinweises auf die Patenterteilung: **06.11.2013**
(21) Anmeldenummer: 07019070.7
(22) Anmeldetag: 27.09.2007
(51) Int. Cl.: A61B 17/88, B25B 15/02, B25B 23/08, A61B 17/00

(54) **Schraubendreher zum Handhaben einer Schraube im menschlichen oder tierischen Körper**
Screwdriver for handling a screw in a human or animal body
Tournevis pour la manipulation d'une vis dans un corps humain ou animal

(30) Priorität: 28.09.2006 DE 102006047674
(43) Veröffentlichungstag der Anmeldung: 02.04.2008
(73) Patentinhaber: Karl Storz GmbH & Co. KG, 78532 Tuttlingen (DE)
(72) Erfinder: Doll, Frank, 78607 Talheim (DE); Walter, Christian, 78576 Emmingen-Liptingen (DE)
(74) Vertreter: Weller, Wolfgang

(56) Entgegenhaltungen:
- CH-A- 368 761
- DE-A1- 3 804 749
- DE-A1- 19 832 303
- US-A- 2 312 869
- US-A- 2 796 099

## Beschreibung

Die Erfindung betrifft einen Schraubendreher zum Handhaben einer Schraube im menschlichen oder tierischen Körper, mit einem Griff, mit einem mit dem Griff fest verbundenen Schaft, an dessen distalem Ende eine Schraube aufnehmbar ist, mit einem den Schaft umgebenden Außenrohr und mit einer Steuerung, um das Außenrohr in unterschiedliche axiale Verschiebepositionen relativ zum Schaft zu bringen, wobei die Steuerung einen Arretiermechanismus aufweist, durch den das Außenrohr in zumindest drei unterschiedlichen axialen Verschiebepositionen relativ zum Schaft dadurch arretierbar ist, dass ein Arretierelement in einer jeweiligen Verschiebeposition in eine jeweilige Arretierstelle eingreift und dort durch eine mechanische Sperre an einem weiteren axialen Vorschub gehindert ist, die jeweils nur dadurch überwindbar ist, dass der Schaft und das Außenrohr relativ zueinander um eine Längsachse verdreht werden, und wobei eine Führung zwischen den zumindest zwei Arretierstellen ausgebildet ist, deren Bahn eine fest vorgegebene Wegstrecke darstellt, die das Arretierelement von einer Arretierstelle zur nächsten Arretierstelle durchläuft.

Ein Schraubendreher mit diesen konstruktiven Merkmalen ist aus der CH 368 761 A bekannt und dient zum Anbringen und Lösen von Schlitzkopfschrauben namentlich an schwer zugänglichen Stellen von Maschinenteilen, Werkstücken etc.

Aus dem medizinischen Bereich ist ein Schraubendreher aus der DE 198 32 303 A1 bekannt. Derartige Schraubendreher dienen auf dem Gebiet der Medizin dazu, um Schrauben in einen menschlichen oder tierischen Körper einzudrehen oder wieder zu entfernen. Der mit dem Griff fest verbundene Schaft dient dazu, mit dem Kopf der Schraube in Eingriff zu treten, um diese drehen zu können. Das den Schaft umgebende Außenrohr ist in unterschiedliche axiale Verschiebepositionen bringbar, um die Handhabung der Schraube im menschlichen Körper zu erleichtern.

In einer Stellung ist das Außenrohr soweit axial nach distal verschoben, dass der Schaft samt der daran angebrachten Schraube vollständig in dem Außenrohr aufgenommen ist. In dieser Stellung kann der Zusammenbau aus Schraubendreher und Schraube minimal-invasiv beispielsweise über einen Trokar in den Körper eingeführt und an die Stelle herangeführt werden, an der die Schraube eingedreht werden soll.

In einer weiteren Stellung ist das Außenrohr etwas nach proximal zurückgezogen, so dass zumindest die Spitze der Schraube über das Außenrohr hinausschaut.

In dieser Stellung kann der Operateur mit der Spitze genau die Eindrehstelle anzielen.

In einer weiteren dritten Stellung kann das Außenrohr soweit zurückgezogen werden, dass die Schraube und auch der mit ihr verbundene Schaft distal vollständig über das Außenrohr hinausschauen. In diesem Zustand kann dann die Schraube vollständig eingedreht werden. Um den Schraubendreher nach einem chirurgischen Eingriff reinigen zu können, ist dieser zerlegbar, wozu das Außenrohr in eine weitere Stellung gebracht werden kann, in der dieses dann vom Griff abgenommen werden kann.

Ein aus der CH 368761 bekannter Schraubendreher weist einen Schaft auf, an dessen distalem Ende eine Klemmhülse angeordnet ist, die durch eine Drehbewegung für ein Einklemmen bzw. Lösen des Kopfes einer am distalen Ende des Schraubendreherschaftes angeordneten Schraube sorgt. Bei dieser Drehbewegung bewegt sich die Klemmhülse längs einer schräg ansteigenden Schlitzbahn in axialer Richtung. Dieser Einspannmechanismus kann durch eine separate, zweite entlang des Schaftes bewegbare zweite Hülse deaktiviert werden. Dazu ist die Klemmhülse an der zweiten Hülse angeordnet. In dieser Ausgestaltung besitzt die Klemmhülse hierfür eine Führung, in der ein Stift der zweiten Hülse aufgenommen ist. Die zweite Hülse ist schließlich mit Hilfe eines weiteren Stifts, der sich in einer Arretiervorrichtung zwischen zwei Arretierpositionen bewegt, an einer dritten Hülse am Schaft angeordnet. In der dritten Hülse ist eine lineare Führung für den weiteren Stift ausgespart, die zwei endseitige Arretierpositionen aufweist. Dies wird dadurch erreicht, dass die dritte Hülse etwas verdreht wird und der weitere Stift in eine seitlich versetzte Ausnehmung der linearen Führung eintritt.

Bei dem eingangs erwähnten Schraubendreher der DE 198 32 303 A1 sind zwei relativ zueinander verschiebbare Rohrschäfte vorhanden, nämlich der bereits erwähnte Außenrohrschaft und ein in diesem aufgenommene Innenrohrschaft. Der Innenrohrschaft trägt distal eine Spannzange, die dazu dient, um die Schraube festzuhalten, damit diese nicht von dem inneren festen Schaft abfällt. Die Spannzange besteht aus einem radial etwas gespreizten geschlitzten Endabschnitt des Innenrohres. Diese radial gespreizten Endabschnitte werden radial nach innen gedrückt, wenn der Außenrohrschaft über den Innenrohrschaft im Bereich der Spannzange geschoben wird.

Die Steuerung der Relativbewegung von Innenrohrschaft und Außenrohrschaft untereinander und die Bewegung dieser beiden relativ zum inneren Schaft, der die Schraube drehen soll, ist sehr kompliziert aufgebaut. Beide Schäfte weisen an ihrem distalen Ende radial vorspringende Stifte auf, die beim Montieren in jeweils ein Steuerelement eingreifen. Die beiden Steuerelemente sind um eine gemeinsame quer zur Längsachse des Schraubendrehers verlaufende Schwenkachse schwenkbar und weisen Steuerkurven auf, die mit den radial vorspringenden Zapfen der Schäfte in Verbindung stehen. Durch entsprechende Konturierung findet die unterschiedliche Relativverschiebung der beiden Schäfte statt. Das axiale Verschieben der beiden Rohrschäfte erfolgt über einen hülsenförmigen Schieber, in dem die Steuerelemente verschwenkbar aufgenommen sind. Der Schieber selbst ist axial verschiebbar und weist axial voneinander, in einer Linie ausgerichtete, beabstandete Arretierstellen auf, in die federbelastete Kugeln einrasten können. Zum Steuern der unterschiedlichen Relativpositionen der Rohrschäfte zwischen den Stellungen, in denen die Schraube vollkommen in dem Außenschaft eingezogen ist, die Spitze etwas über diesen hinausschaut und die Stellung, in der die Schraube vollständig aus dem Außenrohr ausgeschoben ist, erfolgt ausschließlich durch eine lineare Bewegung des Schiebers.

Da die Arretierstellungen nur als geringe Vertiefungen an der Innenseite des Schiebers ausgebildet sind, in denen jeweils federbelastete Kugeln einrasten, kann es vorkommen, dass eine mittlere Raststelle übersprungen wird, und der Schieber beispielsweise von der axial am weitesten zurückgezogenen Position, in der die Schraube vollkommen in dem Außenrohr aufgenommen ist, schon bereits in die maximal vorgeschobene Stellung bewegt werden kann, in der die Schraube vollständig aus dem Außenrohr ausgestoßen wird.

Anders ausgedrückt, beim kräftigen Bewegen des Schiebers, kann eine mittlere Raststellung überfahren werden. Das hat die fatale Folge, dass die Schraube schon aus dem Außenrohr vollständig ausgestoßen wird, bevor diese an ihre Stelle angezielt und die Spitze schon etwas eingedreht ist. Das beinhaltet die Gefahr, dass die Schraube im Körper verloren geht, ohne dass sie eingedreht werden kann.

Darüber hinaus ist der Aufbau des Schraubendrehers mit dem Innenschaft und den beiden relativ zueinander verschiebbaren Außenrohren relativ kompliziert. Auch die Steuerung über die in dem Schieber verschwenkbaren Steuerelemente ist sehr kompliziert und bietet zahlreiche Bakteriennischen, die beim Reinigen Probleme bereiten.

In der DE 198 32 303 A1 ist an einer Stelle erwähnt, dass der Schieber nicht nur axial verschiebbar ist, sondern auch einen Z-förmigen Verlauf einschlagen kann. Dazu ist der Schieber über einen ersten Abschnitt axial verschiebbar, über einen zweiten Abschnitt nur verdrehbar und über einen dritten wiederum nur axial verschiebbar. Dies kann dazu ausgenutzt werden, eine Sperre für den Schieber zu schaffen. Diese Sperre ist dazu vorgesehen, beim Montieren der beiden Rohrschäfte im Griff dafür zu sorgen, dass diese nicht versehentlich durch axiales Verschieben des Schiebers sich lösen. Diese Sperre ist also eine Montagesperre, die dafür sorgt, dass ein axiales Verschieben des Schiebers, wie er im eigentlichen Gebrauch erfolgt, nicht dazu führen kann, dass sich der Griff von den beiden Schäften löst.

Für die Handhabungsperson, also den Arzt, der den Schraubenzieher handhabt, eröffnen sich aber ausschließlich axiale Verschiebepositionen während der Handhabung mit dem bereits erwähnten Nachteil, dass bei zu kräftigem Vor- oder Rückschieben des Schiebers eine Zwischenraststellung überlaufen wird.

Es ist daher Aufgabe der vorliegenden Erfindung, einen Schraubendreher der eingangs genannten Art dahingehend weiterzubilden, dass das Außenrohr in unterschiedliche Verschiebepositionen zum Schaft gebracht werden und in diesen Verschiebepositionen arretiert werden kann, ohne dass eine oder mehrere Arretierstellungen durch axiale Verschiebebewegungen überfahren werden können.

Erfindungsgemäß wird die Aufgabe dadurch gelöst, dass die Arretierstellen als Vertiefungen in der Bahn ausgebildet sind.

In diese Vertiefungen können die Arretierelemente einrasten. Das entsprechende Klickgeräusch zeigt dem Operateur an, dass er eine Arretierstelle erreicht hat.

Diese Maßnahmen haben den erheblichen Vorteil, dass durch die Ausbildung der Steuerung mit einer Sperre gegen einen weiteren axialen Vorschub das Überspringen einer oder mehrerer Arretierstellen vollkommen ausgeschlossen ist.

Um das mechanische Hindernis zu überwinden, muss der Operateur den Schaft und das Außenrohr relativ zueinander um eine Längsachse verdrehen. Dadurch ist sichergestellt, dass kein unkontrolliertes Überspringen einer oder mehrerer Arretierstellen beim axialen Verschieben vorkommt, so dass das Einführen und das Eindrehen der Schraube in dem menschlichen oder tierischen Körper insgesamt atraumatisch durchgeführt werden kann. Dies hat auch den Vorteil, dass die Handhabung des Schraubendrehers für den Operateur einfach ist und die Betätigung ohne besondere Aufmerksamkeit durchgeführt werden kann. Er braucht kein Feingefühl zu entwickeln, ob er sich in einer von mehreren möglichen axialen Raststellungen befindet, sondern er kann so lange axial verschieben, bis er an die Sperre bzw. das mechanische Hindernis trifft.

Die Führung für das Arretierelement hat den Vorteil, dass der Weg des sich von einer Arretierstelle zur nächsten Arretierstelle bewegenden Arretierelements durch die Führung bestimmt ist, d.h. dass das Arretierelement nur entlang der Bahn bewegbar ist.

Dadurch, dass die Bahn, d. h. eine fest vorgegebene Wegstrecke, die das Arretierelement von einer Arretierstelle zur nächsten Arretierstelle durchläuft, um den Winkel α aus der Längsachse abweicht, wird erreicht, dass das Arretierelement nicht durch eine axiale Bewegung, sondern eine von der axialen Bewegung abweichende Bewegung von einer Arretierstelle zur nächsten Arretierstelle gebracht wird.

In einer weiteren Ausgestaltung der Erfindung beträgt der Winkel α vorzugsweise 30° bis 90°, insbesondere etwa 90°.

Diese Maßnahme hat den Vorteil, dass durch den Winkel α im oben genannten Winkelbereich ein besonders sicheres mechanisches Hindernis gegen weiteren axialen Vorschub erzielt wird.

In einer weiteren Ausgestaltung der Erfindung ist die Bahn als eine Nut im Außenrohr ausgebildet.

Diese Maßnahme hat den Vorteil, dass die Nut eine mechanisch einfache Führung für das Arretierelement darstellt, die auch einfach herstellbar ist.

In einer weiteren Ausgestaltung der Erfindung ist das Arretierelement als ein Stift ausgebildet.

Diese Maßnahme hat den Vorteil, dass ein konstruktiv einfaches Arretierelement geschaffen wird, durch das eine ausreichend feste Verrastung in den Arretierstellen bewirkt wird.

In einer weiteren Ausgestaltung der Erfindung ist der Stift federbelastet.

Durch die Federbelastung ist eine definierte Reibung zwischen Arretierelement und Bahn gegeben, die dem Operateur ein Feedback über seine Handhabung gibt.

Darüber hinaus wird der Stift über die Federbelastung in die Arretierstellen hineingedrückt, so dass eine ausreichende Verrastung bewerkstelligt werden kann.

In einer weiteren Ausgestaltung der Erfindung springt das Arretierelement von einer Innenseite des Griffs vor.

Diese Maßnahme hat den Vorteil, dass diese Bauelemente das Ergreifen und Handhaben des Griffs nicht stören, und dass mit diesen beiden Bauelementen die Steuerung bewerkstelligt werden kann.

In einer weiteren Ausgestaltung der Erfindung sind die Arretierstellen an einem proximalen Ende des Außenrohrs angeordnet.

Diese Maßnahme hat den Vorteil, dass der genannte Arretier- und Steuermechanismus am proximalen, nicht in den Körper eindringenden Abschnitt des Schraubendrehers angeordnet ist, somit vor Kontaminationen durch Körperflüssigkeiten erheblich geschützt ist.

In einer weiteren Ausgestaltung der Erfindung ist ein proximaler Endabschnitt des Außenrohrs um dessen Längsachse drehbar.

Diese Maßnahme hat den Vorteil, dass im verrasteten Zustand der Griff samt Schaft und Schraube sowie der mit dem Griff verrastete Abschnitt des Außenrohrs gedreht werden kann.

In einer weiteren Ausgestaltung der Erfindung ist das Außenrohr abnehmbar mit dem Griff verbunden.

Diese Maßnahme hat den Vorteil, dass die Teile des Schraubendrehers aufgrund seiner Zerlegbarkeit separat reinigbar sind. Auf diese Weise erfüllt der erfindungsgemäße Schraubendreher die an medizinische Instrumente im Allgemeinen gestellten hohen Anforderungen an die Desinfizier- bzw. Reinigbarkeit solcher Instrumente.

In einer weiteren Ausgestaltung der Erfindung ist der proximale Endabschnitt des Außenrohrs mit einer Kupplung verbunden, die für ein Drehen des Außenrohrs nach rechts oder nach links freilaufend ist.

Diese Maßnahme hat den Vorteil, dass durch die Kupplung je nachdem, ob eine Schraube ein- oder ausgedreht werden soll, eine kraftschlüssige Verbindung zwischen Griff und Außenrohr in einer Drehrichtung geschaffen und gelöst wird, so dass eine Schraube durch den dauernd ergriffenen Griff ein- bzw. ausgedreht werden kann.

In einer weiteren Ausgestaltung der Erfindung sind vier Arretierstellen vorhanden.

Diese Maßnahme hat den Vorteil, dass das Außenrohr in vier unterschiedlichen Verschiebepositionen zu dem Schaft arretiert werden kann.

In einer weiteren Ausgestaltung der Erfindung ist von dem proximalen Ende des Außenrohrs bis zur ersten Arretierstelle eine Nut ausgebildet, die an ihrem proximalseitigen Ende offen ist.

Diese Maßnahme hat den Vorteil, dass mittels der offenen Nut sich das Außenrohr in besonders einfach zu bedienender Weise mit dem Griff verbinden lässt. Dabei wird der federbelastete Stift in das offene Ende der Nut eingeschoben. Der Griff wird dann so weit nach proximal bewegt, bis der von der Innenseite des Griffs vorspringende Stift in die erste Arretierstelle eingreift.

In einer weiteren Ausgestaltung der Erfindung ist von der letzten Arretierstelle bis zum proximalen Ende des Außenrohrs ebenfalls eine Nut ausgebildet, die an ihrem proximalseitigen Ende offen ist.

Diese Maßnahme hat den Vorteil, dass durch die Nut das Außenrohr und der Griff voneinander getrennt werden können, indem der Stift von der letzten Arretierstelle entlang der Nut bis ihren offenem Ende geführt wird.

In einer weiteren Ausgestaltung der Erfindung ist die Nut stufenartig ausgebildet.

Diese Maßnahme hat den Vorteil, dass durch eine derartige Ausbildung der Nut die Führung exakt und vorbestimmt ist und dem Operateur ein gutes Gefühl vermittelt, in welchem Verschiebezustand die Vorrichtung ist.

Es versteht sich, dass die vorstehend genannten und nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Die Erfindung wird nachfolgend anhand eines ausgewählten Ausführungsbeispiels im Zusammenhang mit den Zeichnungen näher beschrieben und erläutert. Es zeigen:
- Fig. 1: eine perspektivische Seitenansicht eines erfindungsgemäßen Schraubendrehers, wobei der Griff samt Schaft in das Außenrohr eingeschoben ist, diese aber noch nicht miteinander verbunden sind,
- Fig. 2: den Schraubendreher in einer Gesamtdarstellung, teilweise im Längsschnitt entlang einer Längsachse, wobei ein Arretierelement in eine erste Arretierstelle eingreift, in der die Schraube völlig vom Außenrohr umgeben ist,
- Fig. 3: eine der Darstellung von Fig. 2 vergleichbare Darstellung, wobei das Arretierelement in eine dritte Arretierstelle eingreift, in der die Schraube frei liegt,
- Fig. 4: eine der Darstellung von Fig. 2 vergleichbare Darstellung, wobei das Arretierelement in eine vierte Arretierstelle eingreift, in der der distale Endbereich des Schaftes aus dem Außenrohr ausgeschoben ist, und
- Fig. 5: eine plane Abwicklung der Führung eines weiteren Ausführungsbeispiels eines Schraubendrehers.

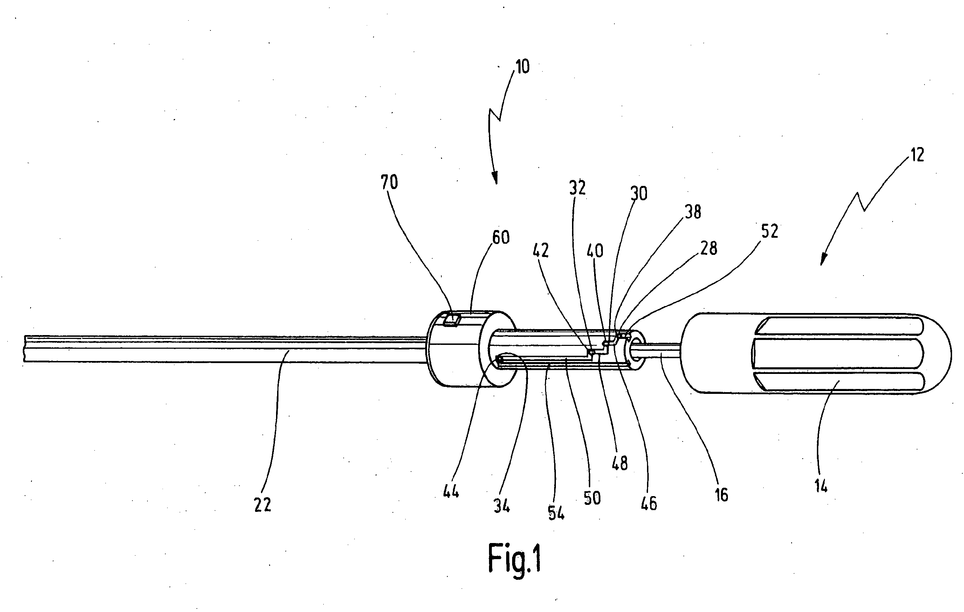

In Fig. 1 ist ein mit dem allgemeinen Bezugszeichen 10 versehener Schraubendreher dargestellt. Der Schraubendreher 10 dient zum Handhaben einer Schraube in einem menschlichen oder tierischen Körper.

Der Schraubendreher 10 weist an einem proximalen Ende 12 einen Griff 14 auf. Mit dem Griff 14 ist ein Schaft 16 fest verbunden, der in Fig. 1 nur teilweise ersichtlich ist. Der Schaft 16 ist langerstreckt und weist eine Länge von etwa 30 cm auf.

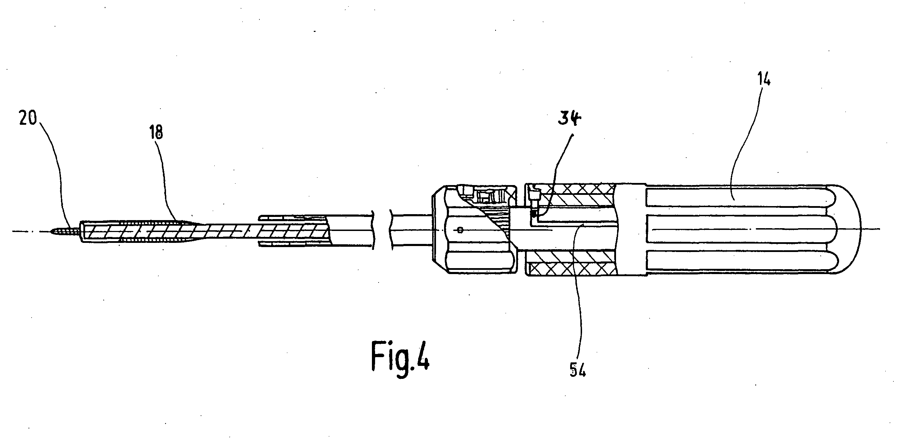

Der Schaft 16 weist an seinem distalen Ende einen Kopf 18 auf, wie es insbesondere aus einer Darstellung von Fig. 4 ersichtlich ist. An dem Kopf 18 ist eine Schraube 20 in einer Halterung aufgenommen.

Der Schraubendreher 10 weist ferner ein Außenrohr 22 auf. Das Außenrohr 22 ist mit dem Griff 14 abnehmbar verbunden. In der Darstellung von Fig. 1 sind die beiden Elemente 14 und 22 nicht miteinander verbunden. Eine Steuerung dient zur definierten Verschiebung des Außenrohrs 22 relativ zum Schaft 16.

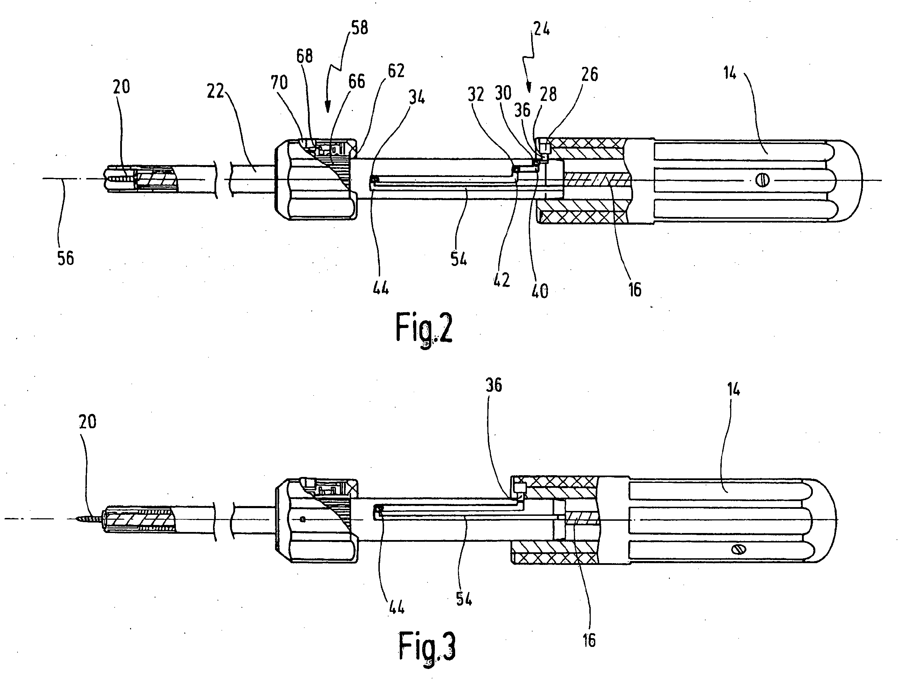

Dazu weist der Schraubendreher 10 einen Arretiermechanismus 24 auf (Fig. 2). Der Arretiermechanismus 24 besteht aus zwei Elementen, nämlich aus einem Arretierelement 26 und mehreren Arretierstellen 28, 30, 32, 34. Das Arretierelement 26, das aus der Darstellung von Fig. 2 ersichtlich ist, springt von einer Innenseite des Griffs 14 vor.

Das Arretierelement 26 ist in diesem Ausführungsbeispiel als ein federbelasteter Stift 36 ausgebildet.

In diesem Ausführungsbeispiel weist der Schraubendreher 10 vier Arretierstellen 28, 30, 32, 34 auf.

Zwischen den Arretierstellen 28, 30, 32, 34 ist eine Führung 46 ausgebildet, deren Bahn 48, d.h. eine fest vorgegebene Wegstrecke, die das Arretierelement 26 von einer Arretierstelle zur nächsten Arretierstelle durchläuft, als eine Nut 50 ausgebildet ist.

In diesem Ausführungsbeispiel ist die Nut 50 stufenartig ausgebildet und zwar derart, dass zwischen zwei benachbarten Arretierstellen 28, 30, 32, 34 jeweils eine rechtwinklige Stufe angeordnet ist. Die Arretierstellen 28, 30, 32, 34 sind als Vertiefungen 38, 40, 42, 44 in der Bahn 48 ausgebildet.

Von dem proximalen Ende des Außenrohrs 22 bis zu der ersten Arretierstelle 28 ist eine Nut 52 ausgebildet, die an ihrem proximalseitigen Ende offen ist. Diese Nut 52 dient dazu, den Griff 14 von proximal auf das Außenrohr 22 aufschieben zu können.

Von der vierten Arretierstelle 34 bis zum proximalen Ende des Außenrohrs 22 ist ebenfalls eine Nut 54 ausgebildet, die auch an ihrem proximalseitigen Ende offen ist. Diese Nut 54 dagegen dient dazu, den Griff 14 und das Außenrohr 22 voneinander trennen zu können. Das Verbinden und das Trennen der beiden Elemente 14, 22 wird später näher beschrieben.

Der proximale Endabschnitt des Außenrohrs 22, an dem die Arretierstellen 28, 30, 32, 34 und die Führung 46 ausgebildet sind, ist um eine Längsachse 56 drehbar. Ein distaler Abschnitt des Außenrohrs 22 ist starr.

Der drehbare Abschnitt des Außenrohrs 22 ist mit einer Kupplung 58 verbunden, die nach rechts bzw. nach links freilaufend ist. Die Kupplung 58 ist in einem Gehäuse 60 angeordnet, das distalseitig mit dem starren Abschnitt des Außenrohrs 22 fest verbunden ist, wie es aus der Darstellung von Fig. 2 hervorgeht.

Die Kupplung 58 weist ein Element 62 auf, an dessen Außenseite Rastkerben 66 eingeschnitten sind, in denen eine Raste 68 zum Eingriff kommt.

Die Raste 68 steht mit einem Knopf 70, der an dem Gehäuse 60 angeordnet ist, derart in Wirkverbindung, dass ein Betätigen des Knopfes 70 ein Abheben der Raste 68 von den Rastkerben 66 bewirkt. Somit kommt es zur Entriegelung der Kupplung 58, wodurch auch ein Drehen des Außenrohrs 22 ermöglicht wird.

Die Handhabung und Betätigung des erfindungsgemäßen Schraubendrehers 10 anhand der zuvor beschriebenen Steuerung soll im Ablauf von Fig. 2 bis 4 kurz erläutert werden.

Um das Außenrohr 22 mit dem Griff 14 zu verbinden, wird der federbelastete Stift 36 in die Nut 52 durch das proximal offene Ende der Nut 52 eingeführt.

Durch ein axiales Bewegen des Griffs 14 wird der von der Innenseite des Griffs 14 vorspringende federbelastete Stift 36 entlang der Nut 52 so weit in Richtung distal geschoben, bis er die erste Arretierstelle 28 erreicht. Der Stift 36 greift in die Vertiefung 38 ein und wird dadurch in der ersten Arretierstelle 28 arretiert. Ein weiteres axiales Vorschieben ist nicht möglich, da die Nut 52 über ein eine 90°-Stufe bzw. Abwinklung in den nächsten axial verlaufenden Abschnitt der Bahn 48 übergeht. Die distalseitige Flanke dieser Stufe bzw. Abwinklung stellt somit ein mechanisches Hindernis vor weiterem axialen Vorschub des Griffes 12 dar.

Die Länge des Außenrohrs 22 und des Schaftes 16 sind so aufeinander abgestimmt, dass in dieser ersten Arretierstellung die an dem Kopf 18 des Schaftes 16 angebrachte Schraube 20 vollständig in dem Außenrohr 22 aufgenommen ist, wie es aus der Darstellung von Fig. 2 ersichtlich ist.

In dieser Arretierstellung wird der erfindungsgemäße Schraubendreher 10 mit der in dem Außenrohr 22 aufgenommenen Schraube 20 direkt oder über einen Trokar in das Operationsgebiet eingeführt, ohne dass die Schraube 20 von dem Schraubendreher 10 abfallen kann.

Um den Stift 36 von der ersten Arretierstelle 28 zur zweiten Arretierstelle 30 zu bringen, muss der Griff 14 relativ zum Außenrohr 21 verdreht werden. Dies ist durch die als Nut 50 ausgebildete Führung 46 zwangsgeführt und die Stufe sperrt eine durchgehende Linearverschiebung, ohne ein Drehen.

Zuerst wird der Griff 14 um die Längsachse 56 gedreht, wodurch der Stift 36 aus der ersten Arretierstelle 28 ausrastet und entlang eines ersten Querabschnitts der Stufe geführt wird. Um den Stift 36 entlang eines zweiten axialen Abschnitts der Stufe zu führen, wird der Griff 14, nachdem der Stift das Ende des ersten Abschnitts erreicht hat, axial nach vorne geschoben. Der Griff 14 kann so weit axial nach vorne geschoben werden, bis der Stift 36 in die zweite Arretierstelle 30 gebracht wird. Durch ein Eingreifen des federbelasteten Stiftes 36 in die Vertiefung 40 wird der Griff 14 in der zweiten Arretierstelle 30 relativ zu dem Schaft 16 arretiert.

In dieser Arretierstellung steht die Schraube 20 teilweise über dem Außenrohr 22 hervor, somit kann mit der vorstehenden Spitze der Schraube 20 der Zielort, an dem sie eingedreht werden soll, anvisiert werden.

Sobald die Schraube 20 angesetzt worden ist, wird der Griff 14 erneut betätigt.

Das Überbringen des Stiftes 36 von der zweiten Arretierstelle 30 zur dritten Arretierstelle 32 erfolgt ebenfalls zuerst durch das Drehen und eine darauf folgende axiale Bewegung des Griffs 14. Dabei rastet der Stift 36 zunächst aus der zweiten Arretierstelle aus, läuft durch die zweite 90° Stufe bzw. Abwinklung bis dieser in die dritte Arretierstelle 32 einrastet.

In der dritten Arretierstellung, die aus der Darstellung von Fig. 3 ersichtlich ist, steht die Schraube 20 vollständig vor dem Außenrohr 22 vor.

In dieser Arretierstellung kann die sich an dem Zielort befindliche Schraube 20 vollständig in den Knochen eingedreht werden. Das Eindrehen der Schraube wird durch ein Drehen des Griffes 14 in Richtung nach rechts erfolgen. Beim Eindrehen der Schraube 20 ergreift eine Hand des Operateurs den Griff 14.

Nachdem die Schraube 20 in den Knochen vollständig eingedreht worden ist, wird der Griff 14 erneut betätigt, um den Stift 36 von der dritten Arretierstelle 32 zur vierten Arretierstelle 34 zu bringen. Auch dies erfolgt wieder über eine kombinierte Dreh/Schiebebewegung.

In der vierten Arretierstelle 34, die aus der Darstellung von Fig. 4 ersichtlich ist, in die der Stift 36 auf die zuvor beschriebene Art und Weise gebracht wird, kann die zwischenzeitlich eingedrehte Schraube 20 vom Kopf 18 gelöst werden.

Nach dem Freigeben der Schraube 20 wird der Schraubenzieher 10 aus dem Körper des Patienten entfernt.

Durch ein erneutes Drehen des Griffes 14 kann der Stift 36 aus der vierten Arretierstelle 34 ausgerastet werden und entlang der Nut 54 bis zu ihrem offenen Ende nach proximal verschoben werden. Dadurch werden das Außenrohr 22 und der Griff 14 voneinander getrennt. In einem zerlegten Zustand kann der Schraubendreher 10 gründlich gereinigt werden.

Je nachdem, wie die Kupplung 58 geschaltet ist, kann die Schraube nach rechts eingedreht werden und die Kupplung ist nach links freilaufend oder die Schraube kann nach links ausgedreht werden und die Kupplung ist nach rechts freilaufend.

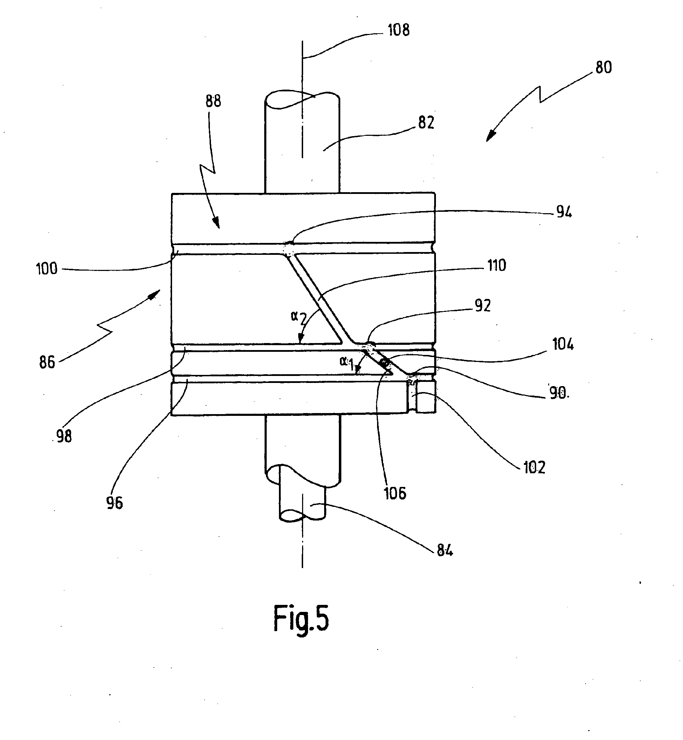

In Fig. 5 ist ein weiteres Ausführungsbeispiel eines Schraubendrehers 80 abschnittsweise dargestellt. Dargestellt ist das Außenrohr 82, in dem der Schaft 84 eingeschoben ist.

In Fig. 5 ist eine plane Abwicklung 86 eines Teils der äußeren zylindrischen Umfangsfläche des Außenrohres 82 dargestellt, in der der Arretiermechanismus 88 ausgebildet ist.

Der Arretiermechanismus 88 weist drei Arretierstellen 90, 92, 94 auf, die jeweils als Vertiefungen in Flanken von axial voneinander beabstandeten umfänglichen Ringnuten 96, 98 und 100 vorhanden sind.

Eine Einführnut 102 erstreckt sich in axialer Richtung, ist zum proximalen Ende hin offen und mündet in der ersten Arretierstelle 90 in der umfänglichen Nut 96.

Die erste umfängliche Ringnut 96 ist über eine um den Winkel α₁ um die Längsachse 108 geneigte Bahn 104 mit der nächsten umfänglichen Ringnut 98 verbunden. Die Bahn 104 geht etwas abseits von der Arretierstelle 90 aus.

Das bedeutet, ein von der Innenseite des hier nicht dargestellten Griffes vorspringendes Arretierelement in Form eines Stiftes 104 trifft, nachdem es zunächst in die Einführnut 102 eingeführt worden ist, auf die Arretierstelle 90 in der ersten umfänglichen Nut 96. Ein weiteres axiales Vorschieben des Griffes bzw. des Stiftes 104 ist erst dann möglich, wenn Schaft 84 bzw. der mit diesem verbundenen Griff und Außenrohr 82 um die Längsachse 108 zueinander verdreht werden. Erst dann kann der Stift 104 in die geneigte Bahn 106 eintreten und bis zur nächsten Arretierstelle 92 vorbewegt werden.

Dies entspricht dem zuvor beschriebenen Übergang, bei dem die Spitze der Schraube etwas aus dem Außenrohr 82 hinausragt, damit die an der einzuschraubenden Stelle angesetzt werden kann.

Um ein weiteres axiales Vorschieben des Stiftes zu ermöglichen, muss erneut der Schaft 84 relativ zum Außenrohr 82 um die Längsachse 108 gedreht werden, damit der Stift 104 dann in die geneigte Bahn 110 eintreten kann und bis zur nächsten Arretierstelle 94 gleiten kann. Auch diese Bahn 110 ist um einen Winkel α₂ aus der Längsachse 108 geneigt.

In den dargestellten Ausführungsbeispielen sind die Winkel α₁ und α₂ unterschiedlich, sie können mehr oder weniger geneigt sein, sie können auch beide gleich sein oder in Form einer Schraubenlinie gewunden sein.

Diese Konstruktion mit den drei voneinander beabstandeten umfänglichen Ringnuten 96, 98, 100 entspricht dem eingangs erwähnten Stand der Technik, wobei hier nun entsprechend erfindungsgemäß die Maßnahmen vorgesehen sind, wonach ein Bewegen des Arretierelements von der einen zur anderen Nut nur über eine relative Drehbewegung zwischen Außenrohr 82 und Schaft 84 ermöglicht ist.

## Patentansprüche

1. Schraubendreher zum Handhaben einer Schraube (20) im menschlichen oder tierischen Körper, mit einem Griff (14), mit einem mit dem Griff (14) fest verbundenen Schaft (16, 84), an dessen distalem Ende eine Schraube (20) aufnehmbar ist, mit einem den Schaft (16) umgebenden Außenrohr (22, 82) und mit einer Steuerung, um das Außenrohr (22, 82) in unterschiedliche axiale Verschiebepositionen relativ zum Schaft (16, 84) zu bringen, wobei die Steuerung einen Arretiermechanismus (24) aufweist, durch den das Außenrohr (22, 82) in zumindest drei unterschiedlichen axialen Verschiebepositionen relativ zum Schaft dadurch arretierbar ist, dass ein Arretierelement (26) in einer jeweiligen Verschiebeposition in eine jeweilige Arretierstelle (28, 30, 32, 34; 90, 92, 94) eingreift und dort durch eine mechanische Sperre an weiterem axialen Vorschub gehindert ist, die jeweils nur dadurch überwindbar ist, dass der Schaft (16, 84) und das Außenrohr (22, 82) relativ zueinander um eine Längsachse (56, 108) verdreht werden, und wobei eine Führung (46) zwischen den zumindest drei Arretierstellen (28, 30, 32, 34; 90, 92, 94) ausgebildet ist, deren Bahn (48, 106, 110) eine fest vorgegebene Wegstrecke darstellt, die das Arretierelement (26) von einer Arretierstelle (28, 30; 90) zur nächsten Arretierstelle (30, 32; 92) durchläuft, **dadurch gekennzeichnet, dass** die Arretierstellen (28, 30, 32, 34) als Vertiefungen (38, 40, 42, 44) in der Bahn (48) ausgebildet sind.

2. Schraubendreher nach Anspruch 1, **dadurch gekennzeichnet, dass** das Arretierelement (26) in der Führung (46) läuft, deren Bahn (48, 106, 110) von einer Arretierstelle (28, 30, 32; 90, 92, 94) zur nächsten Arretierstelle (30, 32, 34) um einen Winkel α aus der Längsachse (56) abweicht.

3. Schraubendreher nach Anspruch 2, **dadurch gekennzeichnet, dass** der Winkel α vorzugsweise 30° bis 90°, insbesondere etwa 90° beträgt.

4. Schraubendreher nach einem der Ansprüche 1 bis 3, **dadurch gekennzeichnet, dass** die Bahn (48, 100, 110) als eine Nut (50) im Außenrohr (22) ausgebildet ist.

5. Schraubendreher nach einem der Ansprüche 1 bis 4, **dadurch gekennzeichnet, dass** das Arretierelement (26) als ein Stift (36, 104) ausgebildet ist.

6. Schraubendreher nach Anspruch 5, **dadurch gekennzeichnet, dass** der Stift (36) federbelastet ist.

7. Schraubendreher nach einem der Ansprüche 1 bis 6, **dadurch gekennzeichnet, dass** das Arretierelement (26) von einer Innenseite des Griffs (14) vorspringt.

8. Schraubendreher nach einem der Ansprüche 1 bis 7, **dadurch gekennzeichnet, dass** die Arretierstellen (28, 30, 32, 34) an einem proximalen Ende des Außenrohrs (22) angeordnet sind.

9. Schraubendreher nach einem der Ansprüche 1 bis 7, **dadurch gekennzeichnet, dass** ein proximaler Endabschnitt des Außenrohrs (22) um die Längsachse (56) drehbar ist.

10. Schraubendreher nach einem der Ansprüche 1 bis 9, **dadurch gekennzeichnet, dass** das Außenrohr (22) abnehmbar mit dem Griff (14) verbunden ist.

11. Schraubendreher nach Anspruch 9, **dadurch gekennzeichnet, dass** der drehbare proximale Endabschnitt des Außenrohrs (22) mit einer Kupplung (58) verbunden ist, die für ein Drehen des Außenrohrs (22) nach rechts oder links freilaufend ist.

12. Schraubendreher nach einem der Ansprüche 1 bis 11, **dadurch gekennzeichnet, dass** vier Arretierstellen (28, 30, 32, 34) vorhanden sind.

13. Schraubendreher nach einem der Ansprüche 1 bis 12, **dadurch gekennzeichnet, dass** von dem proximalen Ende des Außenrohrs (22) bis zur ersten Arretierstelle (28, 90) eine Nut (52, 102) ausgebildet ist, die an ihrem proximalseitigen Ende offen ist.

14. Schraubendreher nach einem der Ansprüche 1 bis 13, **dadurch gekennzeichnet, dass** von der letzten Arretierstelle (34) bis zum proximalen Ende des Auβenrohrs (22) eine Nut (54) ausgebildet ist, die an ihrem proximalseitigen Ende offen ist.

15. Schraubendreher nach Anspruch 4, **dadurch gekennzeichnet, dass** die Nut (50) stufenartig ausgebildet ist.

## Claims

1. Screw driver for handling a screw (20) in a human or an animal body with a handle (14), and with a stem (16, 84) which is permanently connected to the handle (14) and to whose distal end a screw (20) can be fitted, further with an outer tube (22, 82) surrounding the stem (16) and with a control for bringing the outer tube (22, 82) into different axial sliding position relatively to the stem (16), wherein the control has a locking mechanism (24) through which the outer tube (22, 82) can be locked in at least three different axial sliding positions relatively to the stem, wherein a locking element (26) engages in a particular sliding position into a particular locking point (28, 30, 32, 34; 90, 92, 94) and is prevented from moving forward further in the axial direction thereby a mechanical obstruction, which can be overcome only by rotating the stem (16, 84) and the outer tube (22, 82) relative to one another about a longitudinal axis (56, 108), and wherein a guide (46) is provided between the at least three locking points (28, 30, 32, 34; 90, 92, 94), whose path (48, 106, 104) is a predetermined path section through which the locking element (26) passes from one locking point (28, 30; 90) to the next locking point (30, 32; 92), **characterized in that** the locking points (28, 30, 32, 34) are shaped as depressions (38, 40, 42, 44) within the path (48).

2. Screw driver of claim 1, **characterized in that** the locking element (26) runs in the guide (46), which path (48, 106, 110) deviates from the longitudinal axis (56) by an angle α from one locking point (28, 30, 32; 90, 92, 94) to the next locking point (30, 32, 34).

3. Screw driver of claim 1, **characterized in that** the angle α is preferably 30 ° to 90 °, in particular approximately 90°.

4. Screw driver of any one of claims 1 through 3, **characterized in that** the path (48, 106, 110) is embodied as a groove (50) in the outer tube (22).

5. Screw driver of any one of claims 1 through 4, **characterized in that** the locking element (26) is designed as a pin (36, 104).

6. Screw driver of claim 5, **characterized in that** the pin (36) is spring-loaded.

7. Screw driver of any one of claims 1 through 6, **characterized in that** the locking element (26) projects from an inner side of the handle (14).

8. Screw driver of any one of claims 1 through 7, **characterized in that** the locking points (28, 30, 32, 34) are arranged at a proximal end of the outer tube (22).

9. Screw driver of any one of claims 1 through 7, **characterized in that** a proximal end section of the outer tube (22) can be rotated about the longitudinal axis (56).

10. Screw driver of any one of claims 1 through 9, **characterized in that** the outer tube (22) is connected to the handle (14) in a removable fashion.

11. Screw driver of claim 9, **characterized in that** the rotably proximal end section of the outer tube (22) is connected to a coupling (58) which runs freely to the right or to the left for the outer tube (22) to rotate.

12. Screw driver of any one of claims 1 through 11, **characterized in that** four locking points (28, 30, 32, 34) are provided.

13. Screw driver of any one of claims 1 through 12, **characterized in that** a groove (52, 102) which is open at its proximal end is formed from the proximal end of the outer tube (22) to the first locking point (28, 29).

14. Screw driver of any one of claims 1 through 13, **characterized in that** a groove (54) which is open at its proximal end is formed from the last locking point (34) to the proximal end of the outer tube (22)

15. Screw driver of claim 4, **characterized in that** the groove (50) is designed in a step-like manner.

## Revendications

1. Tournevis destiné à la manipulation d'une vis (20) dans le corps humain ou le corps d'animaux, comprenant un manche (14) ; une tige (16, 84) reliée rigidement audit manche (14), et par l'extrémité distale de laquelle une vis (20) peut être reçue ; un tube extérieur (22, 82) entourant ladite tige (16) ; et une commande conçue pour amener ledit tube extérieur (22, 82) à différents emplacements axiaux pris par coulissement vis-à-vis de ladite tige (16, 84), ladite commande comportant un mécanisme d'arrêt (24) par lequel ledit tube extérieur (22, 82) peut être arrêté en au moins trois emplacements axiaux différents, pris par coulissement vis-à-vis de ladite tige, du fait qu'un élément d'arrêt (26) pénètre, en un emplacement considéré pris par coulissement, dans une zone respective d'arrêt (28, 30, 32, 34 ; 90, 92, 94) dans laquelle il est empêché de poursuivre son avance axiale par un verrou mécanique dont l'action ne peut être respectivement neutralisée que par rotation mutuelle imprimée à la tige (16, 84) et au tube extérieur (22, 82), autour d'un axe longitudinal (56, 108), sachant qu'il est ménagé, entre les trois zones d'arrêt (28, 30, 32, 34 ; 90, 92, 94) à présence minimale, un guide (46) dont la piste (48, 106, 110) instaure une course fermement préétablie parcourue, par l'élément d'arrêt (26), depuis une zone d'arrêt (28, 30 ; 90) jusqu'à la zone d'arrêt (30, 32 ; 92) successive, **caractérisé par le fait que** les zones d'arrêt (28, 30, 32, 34) sont réalisées sous la forme de creusures (38, 40, 42, 44) pratiquées dans la piste (48).

2. Tournevis selon la revendication 1, **caractérisé par le fait que** l'élément d'arrêt (26) se meut dans le guide (46) dont la piste (48, 106, 110) présente un écart d'un angle α, à partir de l'axe longitudinal (56), depuis une zone d'arrêt (28, 30, 32 ; 90, 92, 94) jusqu'à la zone d'arrêt (30, 32, 34) successive.

3. Tournevis selon la revendication 2, **caractérisé par le fait que** l'angle α mesure, de préférence, de 30° à 90°, et environ 90° en particulier.

4. Tournevis selon l'une des revendications 1 à 3, **caractérisé par le fait que** la piste (48, 106, 110) est réalisée sous la forme d'une rainure (50) façonnée dans le tube extérieur (22).

5. Tournevis selon l'une des revendications 1 à 4, **caractérisé par le fait que** l'élément d'arrêt (26) est réalisé sous la forme d'un téton (36, 104).

6. Tournevis selon la revendication 5, **caractérisé par le fait que** le téton (36) est contraint élastiquement.

7. Tournevis selon l'une des revendications 1 à 6, **caractérisé par le fait que** l'élément d'arrêt (26) est en saillie au-delà d'une face intérieure du manche (14).

8. Tournevis selon l'une des revendications 1 à 7, **caractérisé par le fait que** les zones d'arrêt (28, 30, 32, 34) sont situées à une extrémité proximale du tube extérieur (22).

9. Tournevis selon l'une des revendications 1 à 7, **caractérisé par le fait qu'**une région extrême proximale du tube extérieur (22) peut tourner autour de l'axe longitudinal (56).

10. Tournevis selon l'une des revendications 1 à 9, **caractérisé par le fait que** le tube extérieur (22) est relié au manche (14) de manière amovible.

11. Tournevis selon la revendication 9, **caractérisé par le fait que** la région extrême proximale rotative du tube extérieur (22) est reliée à un accouplement (58) conçu avec course libre, en vue d'une rotation dudit tube extérieur (22) vers la droite ou vers la gauche.

12. Tournevis selon l'une des revendications 1 à 11, **caractérisé par** la présence de quatre zones d'arrêt (28, 30, 32, 34).

13. Tournevis selon l'une des revendications 1 à 12, **caractérisé par le fait qu'**une rainure (52, 102), façonnée depuis l'extrémité proximale du tube extérieur (22) jusqu'à la première zone d'arrêt (28, 90), est ouverte à son extrémité située du côté proximal.

14. Tournevis selon l'une des revendications 1 à 13, **caractérisé par le fait qu'**une rainure (54), façonnée depuis la dernière zone d'arrêt (34) jusqu'à l'extrémité proximale du tube extérieur (22), est ouverte à son extrémité située du côté proximal.

15. Tournevis selon la revendication 4, **caractérisé par le fait que** la rainure (50) est de réalisation étagée.
